# EUROPÄISCHE PATENTSCHRIFT

(11) **EP 1 945 178 B1**
(45) Veröffentlichungstag und Bekanntmachung des Hinweises auf die Patenterteilung: **11.01.2012**
(21) Anmeldenummer: 06819377.0
(22) Anmeldetag: 09.11.2006
(51) Int. Cl.: A61K 6/083

(54) **VERFAHREN ZUR HERSTELLUNG VON IM DENTALBEREICH EINSETZBAREN KOMPOSITEN**
METHOD FOR PRODUCING COMPOSITES THAT CAN BE USED IN DENTISTRY
PROCEDE POUR REALISER DES COMPOSITES DESTINES AU DOMAINE DENTAIRE

(30) Priorität: 11.11.2005 DE 102005053954
(43) Veröffentlichungstag der Anmeldung: 23.07.2008
(73) Patentinhaber: Ivoclar Vivadent AG, 9494 Schaan (LI)
(72) Erfinder: RHEINBERGER, Volker, FL-9490 Vaduz (LI); HAGENBUCH, Konrad, CH-9465 Salez (CH); DELLAFIOR, Petra, A-6806 Feldkirch (AT)
(74) Vertreter: Fitzner, Uwe
(86) Internationale Anmeldenummer: PCT/EP2006/068310
(87) Internationale Veröffentlichungsnummer: WO 2007/054547

(56) Entgegenhaltungen:
- EP-A- 0 270 915
- EP-A- 1 502 571
- DE-A1- 2 000 771
- DE-A1- 3 000 213

## Beschreibung

Diese Anmeldung nimmt die Priorität der DE 10 2005 053 954.8-42 in Anspruch.

Die vorliegende Erfindung betrifft ein Verfahren zur Herstellung von im Dentalbereich einsetzbaren Kompositen, die nach diesem Verfahren hergestellten Komposite als solche und deren Verwendung.

In den letzten Jahrzehnten wurden große Anstrengungen unternommen, die Härte und die Abrasionsfestigkeit von kunststoffbasierten Dentalmaterialien zu erhöhen. Dabei wurden vor allem di- oder polyfunktionelle (Meth)acrylatmonomere (z.B. UDMA oder Bis-GMA) mit anorganischen Füllstoffen gefüllt. Unter di- und polyfunktionellen (Meth)acrylatmonomeren werden Monomere verstanden, die zwei oder mehr polymerisierbare (Meth)acrylatgruppen im Molekül besitzen. Diese werden nachfolgend auch als Vernetzer bezeichnet. In Dentalmaterialien werden üblicherweise Mischungen aus mono- und polyfunktionellen Monomeren mit polymerisationsfähigen Gruppen, vorwiegend auf (Meth)acrylatbasis eingesetzt.

Die Bestrebungen gingen soweit, dass beispielsweise bei direkten Füllungsmaterialien Füllgrade von bis zu über 90 Gew.-% anorganischer Füllstoffanteil erreicht wurden. Der hohe Füllstoffgehalt führt jedoch dazu, dass sich die Komposite nicht mehr optimal auf Hochglanz polieren lassen bzw. war es erforderlich, Mischungen von verschiedenen Füllstoffen einzusetzen. Dies macht eine teilweise recht komplizierte Abstimmung der verschiedenen Füllstoffe hinsichtlich der chemischen Zusammensetzung, der Korngröße sowie der Korngrößenverteilung als auch deren Oberflächenbehandlung nötig. Die teilweise entstehende Oberflächenrauhigkeit alleine führt schon zu einer Erhöhung der Plaque- und Verfärbungsanfälligkeit. Dazu kommt, dass die Übergänge zwischen den harten anorganischen Füllstoffpartikeln und der organischen Matrix, die trotz einer chemischen Oberflächenmodifizierung (meist eine Silanisierung) der Füllstoffpartikel durch hydrolytische Prozesse Mikrospalten bilden können. Dies wiederum kann zu einer vermehrten Plaque- und Verfärbungsanfälligkeit führen. Neben den physikalischen Einflüssen neigen Kunststoffe, die auf polyfunktionellen Monomeren aufgebaut sind, im Vergleich zu (Meth)acrylat-Werkstoffen zu einer verstärkten Plaqueablagerung und Verfärbungsanfälligkeit. Plaque ist ein supragingivaler Zahnbelag, der sich als weiche Schicht auf freiliegenden Zahnoberflächen ablagert und besteht vorwiegend aus Nahrungsresten, Speichelbestandteilen sowie Bakterien und deren Stoffwechselprodukten. Als erster Keim heftet sich Streptococcus sanguis über Lectine auf dem Schmelzoberhäutchen an. Von Streptococcus mutans gebildete extrazelluläre Polysaccharide erhöhen die Adhäsion. Vor allem Streptokokken wandeln Zucker in Milch- u. Brenztraubensäure um, die den Schmelz demineralisieren.

Aus der EP 0 674 896 B1 1 ist ein Dentalmaterial bekannt, das quarternäre Ammoniumverbindungen mit keimtötender bakteriostatischer Wirkung enthält.

Aus der WO 98/48766 sind Dentalmaterialien bekannt, die als antibakterielle Substanz Triclosan enthalten. Nachteilig ist jedoch, dass das Triclosan herausgelöst wird, so dass eine langfristige Wirkung nicht erhalten bleibt.

Aus der WO 2004058193 ist ein Dentalmaterial mit bakteriostatischen und/oder bakteriziden Substanzen bekannt. In dieser Anmeldung werden Wirkstoffe beschrieben, z.B. Taurolidin, welches im Bereich pH 6 bis 7 inaktiv ist und infolge dessen erst bei einer Änderung des pH-Wertes infolge der Tätigkeit der Bakterien aktiviert wird. Nachteilig bei den beschriebenen Dentalmaterialien ist darüber hinaus, dass der Einsatz der bakterientötenden Zusätze das natürliche Bakterienmilieu im Mund des Patienten stören bzw. nachhaltig verändern kann.

Auf dem dentalen Gebiet sind jedoch auch Werkstoffe bekannt, welche weniger stark zu Plaqueablagerungen und Verfärbungen neigen als die eingangs beschriebenen Komposite. Dabei handelt es sich einerseits um keramische Werkstoffe und andererseits um thermoplastische Kunststoffe.

Bei den thermoplastischen Kunststoffen nimmt das Polymethylmethacrylat bei dentalen Anwendungen eine wichtige Rolle ein. Dabei wird das PMMA heute wegen den gestiegenen Anforderungen bezüglich der Abrasionsfestigkeit in erster Linie nur noch als Füllstoff für Prothesenzähne und für Prothesenbasismaterialien in der Totalprothetik sowie als Provisorium in der Kronen und Brückentechnik eingesetzt. Dabei werden unvernetzte oder schwach vernetzte PMMA-Partikel in vernetzerhaltigem Mono-Methylmethacrylat gelöst bzw. zu einem verarbeitbaren Teig gequollen und anschließend polymerisiert. Diese auf PMMA basierenden Materialien zeigen eine sehr gute Plaque- und Verfärbungsresistenz. Ein weiterer Thermoplast mit ebenfalls sehr guten Verfärbungs- und Plaqueresistenz ist Teflon. Die optischen und physikalischen Eigenschaften des Materials erfüllen jedoch nicht die Anforderungen, die an ein Dentalmaterial gestellt werden. Keramische Werkstoffe hingegen erfüllen alle Anforderungen, werden jedoch bedingt durch die aufwendige Verarbeitung und den hohen Preis in erster Line für hochwertigen Zahnersatz verwendet. Komposite dagegen stellen eine leicht zu verarbeitende und preiswertere Alternative dar, weisen jedoch noch Mängel auf, die ihre Einsetzbarkeit gegenüber keramischen Werkstoffen einschränken.

Es ist weiterhin bekannt, dass durch die Einarbeitung von Partikeln aus Polymethylmethacrylat in Monomermischungen die Plaque- und Verfärbungsanfälligkeit stark reduziert werden kann.

Aus der US 4,946,901 sind gefällte Füllstoffe auf (Meth)acrylatbasis bekannt, die eine Korngröße von etwa 5 bis 100 µm haben und in Füllungsmaterialien, Zähnen oder auch in Verblendmaterialien eingesetzt werden können.

Aus der US 4,937,144 (= DE 3820497) sind ferner stark quellende Füllstoffe, die aus Vernetzermonomeren in Lösung gefällt werden, für dentale Anwendungen bekannt. Hier werden Polymere mit einem Vernetzungsgrad von 50 - 100 Gew.-% beschrieben, deren mittlere Korngröße ist 0.01 - 10 µm beträgt. Darin wird das Verhältnis von Vernetzermonomeren zum Quellmittel mit 1:1 bis 1:100 angegeben. Grundsätzlich lassen sich alle Kunststoffe quellen. Der Quellungsgrad eines Kunststoffes ist dabei abhängig vom verwendeten Quellungsmittel, der Quelltemperatur und dem Vernetzungsgrat des Kunststoffes.

Ferner sind aus der EP 0 270 915 Füllstoffe für Dentalmassen bekannt, die durch Fällungspolymerisation aus di- bzw. polyfunktionellen Vernetzermonomeren verschiedener (Meth)acrylate hergestellt werden,. Mit diesen Füllstoffen kann die Plaqueanlagerung bzw. Verfärbungsneigung jedoch nicht verringert werden.

Alle diese Füllstoffe werden so hergestellt, dass direkt bei der Fällung ein feines Pulver anfällt. Bei deren Einarbeitung in Komposite ergeben sich jedoch erhebliche Nachteile hinsichtlich der mechanischen Stabilität. Nachteilig ist weiterhin, dass die so erhaltenen Füllstoffpartikel eine geringe Korngröße aufweisen und somit sehr stark verdickend wirken.

Aus der DE 3000213 ist ein Verfahren zur Herstellung eines vernetzten Polymerisats unter Erhöhung der Quellungsgeschwindigkeit bekannt. Hierbei wird ein partiell vernetztes Co-Polymerisat hergestellt, das zur Erhöhung der Quellungsgeschwindigkeit nachfolgend modifiziert wird.

Als eine Variante der Modifizierung wird eine physikalische Modifizierung in Form von Mahlen, Hochenergiestrahlung oder mechanische Gefügeauflockerung durchgeführt. Die zweite Variante ist der Einbau eines nicht vernetzenden Monomers in das Polymergefüge. Danach erfolgt die Polymerisation des nicht vernetzenden Monomers und ein trockener Füllstoff wird erhalten.

Aus der US 2,013,295 ist die Herstellung von Polymethylmethacrylat als Füllstoff für Prothesenmaterialien bekannt. Hierbei wird zur Modifizierung des Polymethylmethacrylats ein Zusatzstoff hinzugefügt. Dies kann auch eine Flüssigkeit sein, z. B. Triacetin oder auch Weichmacher.

Aus der DE-OS 2000771 ist die Herstellung von Hydrogelen bekannt, die in einer Fällungspolymerisation hergestellt und dann zu verschiedenen weichen und flexiblen Produkten weiterverarbeitet werden. Diese Gele aus teilweise vernetzten und teilweise unvernetzten Polymeren mit Lösungsmittelanteilen werden als stabile Füllstoffe in verschiedenen Anwendungen verwendet.

Die vorliegende Erfindung hat sich nunmehr die Aufgabe gestellt, ein Verfahren zur Herstellung von Kompositen zur Verfügung zu stellen, mit welchem sich polymerisierte Materialien, insbesondere Polymethylmethacrylat besser als bisher in die erforderliche Partikelgröße aufmahlen und in Komposite einarbeiten lassen, ohne dass die mechanischen Eigenschaften nachteilig verändert werden, insbesondere ohne dass eine Versprödung des Materials auftritt.

Gelöst wird diese Aufgabe durch ein Verfahren zur Herstellung von im Dentalbereich einsetzbaren Kompositen, bei welchem
a) in einem Lösungsmittel gelöste mono- und polyfunktionelle (Meth)acrylate zu einem mit dem Lösungsmittel durchquollenen Polymergel polymerisiert werden,
b) das Polymergel vorzerkleinert wird,
c) anschließend ein Reaktivgel hergestellt wird, indem in das Polymergel unter gleichzeitiger Entfernung des Lösungsmittels ein polymerisierbares Monomer oder eine polymersierbare Monomermischung eingearbeitet und
d) aus dem so erhaltenen Reaktivgel ein Dentalkomposit hergestellt wird.

In dem erfindungsgemäßen Verfahren werden demgemäß zunächst ein oder mehrere monofunktionelle Monomere mit einem Vernetzer vermischt. Als Monomere kommt erfindungsgemäß vorzugsweise Methylmethacrylat in Betracht. Ebenfalls eignen sich weitere Monomethacrylatverbindungen wie Ethylmethacrylat oder Isobutylmethacrylat, jedoch nimmt die Verfärbungsresistenz mit zunehmendem Molekulargewicht ab.

Als mehrfunktionale Vernetzer kommen beispielsweise Urethandimethacrylat oder Additionsprodukte aus Methacrylsäure und Bisphenol-A-Diglycidylether (Bis-GMA) in Betracht. Hierbei sind kurzkettige Dimethacrylate mit einem niedrigen MolekularGewicht wie z.B. Ethylenglykoldimethacrylat (MG = 198) nicht geeignet, da sie zu einer Versprödung des Polymerisates führen.

Die mono- und polyfunktionellen (Meth)acrylate werden in einem Lösungsmittel' gelöst. Hierfür kommen vorzugsweise inerte Lösungsmittel in Betracht, d. h. Lösungsmittel, die nicht an der Polymerisation teilnehmen. Dadurch soll sichergestellt werden, dass die Lösungsmittel lediglich physikalisch eingebunden werden, ohne an der Polymerisationsreaktion teilzunehmen. Zu den einsetzbaren Lösungsmitteln gehören z.B. Aceton, Ethanol, Hexan, Toluol, Ethylacetat. Der Siedepunkt des Lösungsmittels sollte im Bereich der Temperatur liegen, bei der der Katalysator (z.B. Peroxide, Peroxocarbonate oder Azoverbindungen) Radikale zur Auslösung der Polymerisationsreaktion bildet.

Die genannten Komponenten werden vorzugsweise in folgenden Mengenverhältnissen eingesetzt:
5 bis 70 Gew.-% monofunktionelle Monomere, vorzugsweise 10% bis 50%, besonders bevorzugt 20 bis 35% Gew.-%
5 bis 70 Gew.-% di- oder polyfunktionelle Monomere, vorzugsweise 10 bis 50%, besonders bevorzugt 25 % Gew.-%
20 bis 70 Gew.-% Lösungsmittel, vorzugsweise 30 bis 60%, insbesondere bevorzugt 50 Gew.-%,
   wobei die einzelnen Komponenten sich jeweils auf 100 Gew.-% addieren.

Erfindungsgemäß ist es besonders bevorzugt, wenn das Verhältnis von mono- und polyfunktionellen (Meth)acrylaten zu Lösungsmittel von 1:3 bis 1:0.75 liegt, vorzugsweise 1:2 bis 1:0.9, ganz besonders bevorzugt 1:1. Die Polymerisation der eingesetzten Monomermischung wird vorzugsweise in Gegenwart eines heisshärtenden Katalysators durchgeführt. Als Katalysatoren kommen insbesondere Dilauroylperoxid, Dibenzoylperoxid, Cumolhydroperoxid, Azo-bis(isobutyronitril), tert.-Butylperoctoat, Düsopropyl-peroxodicarbonat in Betracht.

Das Verfahren der Polymerisation wird so durchgeführt, dass die Mischung bis mindestens auf die Temperatur der Radikalbildung des Katalysators erhitzt wird. Danach startet die Polymerisation der Monomermischung. Besonders vorteilhaft ist es, dieses Verfahren als "Kochen unter Rückfluss" nahe am Siedepunkt des verwendeten Lösungsmittels durchzuführen. Beispielsweise wird bei Einsatz von Aceton ein Siedepunkt von ca. 56 °C für die Durchführung des Verfahrens eingestellt. Hierbei entsteht ein mit dem Lösungsmittel durchquollenes Polymergel. Das auf diese Weise hergestellte Polymergel ist leicht zu zerkleinern.

Bei der Auswahl des Lösungsmittels und der Verdünnung des reaktiven Gemisches ist darauf zu achten, dass die durch die exotherme Reaktion entstehende Wärme abgeführt werden kann. Je nach den verwendeten Monomeren und Lösungsmitteln sowie deren Mischungsverhältnisse ist die Struktur des Polymergels weicher oder fester. Je größer das Verhältnis der polymerisierbaren Monomere zum Lösungsmittel ist, desto fester werden die Polymergele.

Das Polymergel enthält das Monomer-/Lösungsmittel-Gemisch in einem Verhältnis von Monomermischung zu Lösungsmittel von 1:3 bis 0.75 , vorzugsweise 1:2 bis 1:0,9, ganz besonders bevorzugt 1:1.

Nach der Auspolymerisierung der Monomere und dem Durchquellen mit dem Lösungsmittel wird das Polymergel dem nächsten Verfahrensschritt, d. h. der Vorzerkleinerung zugeführt. Die Vorzerkleinerung des Polymergels kann in üblichen Geräten stattfinden. Beispielsweise können marktübliche Zerhacker, z.B. Dirks-Mischer, 2- oder 3-Walzenstühle und ähnliche Geräte in Betracht kommen.

Im Anschluss an die Vorzerkleinerung erfolgt die Herstellung des Reaktivgels, d. h. eine Einarbeitung eines polymerisierbaren Monomers in das mit dem Lösungsmittel noch durchquollenen Polymergel. Zugleich mit der Einarbeitung des polymerisierbaren Monomers wird das Lösungsmittel entfernt, d. h. das Lösungsmittel wird durch polymerisierbares Monomer ersetzt. Im Ergebnis entsteht somit ein neues Gel. Dieses Gel wird im Rahmen der vorliegenden Beschreibung als Reaktivgel bezeichnet.

Hierfür können dem Fachmann bekannte Vorrichtungen eingesetzt werden. Erfindungsgemäß bevorzugt ist der Einsatz eines 3-Walzenstuhls. Dreiwalzenstühle sind Homogenisierungs- und Zerkleinerungsanlagen, wie sie in der Lack- und auch Kosmetikindustrie zur Herstellung von Farbpasten und Cremen eingesetzt werden. Dabei wird die Masse durch drei sich gegenläufig drehende Walzen transportiert. Hierbei wird das Polymergel zerkleinert und das polymerisierbare Monomer zur Herstellung des Reaktivgels eingearbeitet. Durch die Einstellung des Walzenspaltes kann eine sehr präzise Partikelzerkleinerung und Dispergierung erreicht werden. (z.B. Exact Dreiwalzenstuhl der Firma EXACT Vertriebs GmbH, 22851 Norderstedt).

Als polymerisierbare Monomere für die Einarbeitung in das Polymergel sind z. B. polymerisierbare Matrixmonomere, wie z.B. Urethandimethacrylat (UDMA), Additionsprodukte aus Methacrylsäure und Bisphenol-A-Diglycidylethern (Bis-GMA), Carbonatdimethacrylate, Ethylenglycoldimethacrylate oder Epoxidharze geeignet. Ebenfalls eigenen sich Silicon- und Polyesterharze.

Erfindungsgemäß ist es darüber hinaus auch möglich, das vorzerkleinerte Polymergel als eine Vorstufe in hochkonzentrierter Form mit Vernetzermonomeren zu Vorpasten zu verarbeiten, die anschließend in Komposite eingearbeitet werden können. Die erfindungsgemäßen Polymergele lassen sich auch in Monomethacrylate einarbeiten, die beispielsweise in lösungsmittelfreien Dentalüberzugslacken als Reaktivverdünner eingesetzt werden können.

Im Ergebnis wird durch die Einarbeitung der vorzerkleinerten Polymergele in polymerisierbare Monomere eine Paste erhalten, in der das anfängliche Polymergel mit dem reaktiven Monomer zu einem Reaktivgel gequollen ist. D.h., es entsteht ein Gel, das eine polymerisierte Komponente (im Rahmen der Erfindung als Polymergel bezeichnet) und eine nicht polymerisierte Komponente in Form eines polymerisierbaren Monomers enthält. Das entstandene modifizierte Gel (Reaktivgel) ist reaktiv, mit anderen Worten polymerisierbar.

Aus dieser Paste lassen sich sodann im Dentalbereich einsetzbare Komposite herstellen. Dies erfolgt vorzugsweise durch Mischung des Reaktivgels mit einem Matrixmonomer und ggf. Polymerisationsinitiatoren. Als Matrixmonomere sind einsetzbar Urethandimethacrylat (UDMA), Additionsprodukte aus Methacrylsäure und Bisphenol-A-Diglycidylethern (Bis-GMA), Carbonatdimethacrylate, Ethylenglycoldimethacrylate oder Epoxidharze. Ebenfalls eigenen sich Silicon- und Polyesterharze.

Zur Herstellung der Komposite können außerdem weitere anorganische Füllstoffe, beispielsweise SiO₂, Glasfüller, Prepolymere oder organische Füllstoffe eingearbeitet werden. Dabei können die Prepolymere auch einen anorganischen Füllstoff enthalten. Ebenfalls können anorganische Füllstoffe auf Basis von Zirkonoxid oder Mischoxide wie Siliziumdioxid und Aluminiumoxid eingesetzt werden. Auch können Nanopartikel mit behandelter Oberfläche eingearbeitet werden. Dabei sollten speziell die anorganischen Füllstoffe mittel eines funktionellen Silans chemisch oberflächenmodifiziert sein, um einen chemischen Verbund zur polymerisierbaren Monomermatrix herzustellen. Der zu erreichende Füllgrad hängt von der Menge des eingearbeiteten gequollenen Füllstoffes, von der Viskosität des Kompositmonomers und der Korngröße der verwendeten restlichen anorganischen und organischen Füllstoffe ab.

Die erfindungsgemäß hergestellten Komposite lassen sich mit allen bekannten Polymerisationsmethoden aushärten. In Abhängigkeit vom verwendeten Katalysatorsystem bzw. vom Härtungsmechanismus des im Komposit eingesetzten Monomeren, ist die entsprechende Bearbeitungsmethode zu wählen. Einsetzbar sind z.B. lichthärtende Materialien (Photoinitiator), kalthärtende Materialien (z.B. Amin/Peroxid) oder heißhärtende Materialien (z.B. Peroxide) oder kationisch härtbare Materialien.

Die nach dem erfindungsgemäßen Verfahren hergestellten Reaktivgele bzw. Komposite lassen sich vielfältig im Dentalbereich einsetzen. So können sie beispielsweise eingesetzt werden für Zähne, Kompositzemente, Füllungskomposite, Fissurenversiegelungslacken, Charakterisierfarben für die Zahnoberflächen usw.

Die erfindungsgemäß hergestellten Komposite haben erhebliche Vorteile gegenüber den bisher bekannten Dentalkompositen. Insbesondere ist eine deutliche Reduktion der Farbstoffaffinität zu verzeichnen, was zu deutlich reduzierten oberflächlichen Verfärbungen der Zahnrestaurationen führt. Im Vergleich zu den bisher bekannten Prepolymerisaten lassen sich außerdem deutlich feinere Korngrößen erzielen (kleiner als 1 µm). Die Partikel weisen eine unregelmäßige Form auf.

Die meisten handelsüblichen Polymethacrylate liegen dagegen als Kugeln vor. Kugeln eignen sich jedoch nicht optimal als Füllstoff, weil kugelförmige Füllstoffpartikel leicht aus der Oberfläche herausbrechen sobald beim Beschleifen der Äquator des Partikels unterschritten wird.

Der Brechungsindex von reinem Polymethylmethacrylat liegt bei 1,49 und ist somit für die Verwendung in Dentalkompositen gut geeignet. Durch die Wahl von Vernetzern mit höheren oder tieferen Brechungsindizes, die erfindungsgemäß zur Gelherstellung verwendet werden, ist der Brechungsindex anpassbar und optimierbar. Ein weiterer Vorteil ist, dass die aus den erfindungsgemäßen Reaktivgelen erhaltenen Produkte nicht spröde sind. Außerdem lassen sich durch den Einbau von jodierten aromatischen (Meth)acrylatvernetzern hohe Röntgenopazitäten erreichen.

Ferner können die mit dem erfindungsgemäß hergestellten Reaktivgel versehenen Matrixsysteme problemlos mit weiteren anorganischen Füllstoffen verstärkt und gefüllt werden. Ebenso lassen sich mit dem erfindungsgemäßen Verfahren gequollene Reaktivgele auf ausschließlicher Basis reiner Vernetzermonomere herstellen, die zur Reduzierung der Abrasion in Polymethylmethacrylatsystemen eingesetzt werden können.

Die Reaktivgele können weiterhin für Silikonunterfütterung für Prothesen zur Reduktion der Plaque- und Verfärbungsanfälligkeit verwendet werden. Dabei kann das füllstoffhaltige Reaktivgel direkt in einem Silikon hergestellt und auf einem Dreiwalzenstuhl zerkleinert bzw. verrieben werden.

Schließlich können unter Verwendung des Reaktivgels auch die Plaque- und die Verfärbungsanfälligkeit von dentalen lösungsmittelfreien Lacken reduziert werden. Diese Lacke können als Polierersatz z.B. auf Kronen und Brücken oder Totalprothesen eingesetzt werden und können so den Aufwand für den Zahntechniker spürbar reduzieren.

Im Folgenden wird die Erfindung unter Bezugnahme auf die Beispiele näher beschrieben, wobei diese Beispiele die Erfindung nicht limitieren:

### Beispiel 1: Mikrogefüllte Komposite Systeme:

### Schritt 1: Gequollener und vernetzter PMMA (Polymergel)

| | |
|---|---|
| Methylmethacrylat | 100 g |
| UDMA 1 (MG 513) | 100 g |
| Aceton | 200 g |
| Dilauroylperoxid | 2 g |

### Schritt 2: Vorzerkleinerung

Das lösungsmittelhaltige Polymergel wird im Labormixer zerkleinert, anschließend wird der Trockenrückstand bzw. Acetongehalt bestimmt:
Acetongehalt = 42 %

### Schritt 3: Einarbeitung in Kompositmonomer

| | | |
|---|---|---|
| Kompositmonomermischung | 42.5 g | 80% UDMA + 20% Decandioldimethacrylat |
| Gequollenesvernetztes Polymergel | 34.5 g | Acetongehalt 35% = 20 g Trockengewicht |

Auf einem Dreiwalzenstuhl Exact 80 E wird die o. g. Mischung homogenisiert. Der Walzenspalt beträgt einheitlich 10 µm. Nach dem Homogenisieren wird das restliche Lösungsmittel (Aceton) bei 55°C im Vakuum entfernt.

### Schritt 4: Herstellung Komposite

| | | |
|---|---|---|
| Kompositmonomer mit Polymergel | 62.5 g | Aus Schritt 3 |
| Pyrogene Kieselsäure silanisiert | 37.0 g | SiO₂, silanisiert |
| Tert. Butylperoctoat | 0.5 g | |
| Total | 100.0 g | |

Im Laborkneter werden die Kieselsäure und der Katalysator eingearbeitet.

### Aufgeschlüsselte Rezeptur

| | |
|---|---|
| Aliphatisches DMA | 8.4 |
| UDMA 1 | 34.1 |
| Erfindungsgemäßes Polymergel | 20.0 |
| Pyrogene Kieselsäure, silanisiert | 37.0 |
| Tert.-Butylperoctoat | 0.5 |
| Total | 100.0 |

| | |
|---|---|
| DMA | Dimethacrylat (Decandioldimethacrylat) |
| UDMA 1 | Addukt aus TMDI (Trimethylhexamethylendüsocyanat) und HEM (Hydroxyethylmethacrylat) |
| UDMA 2 | Urethandimethacrylat Addukt aus TMXDI (1,3-bis -isocyanato 1-methylenethylenbenzol) und HEMA) |

Beispiele:

### Versuchskomposit 1:

| | |
|---|---|
| UDMA Monomer (UDMA 2) | 34.1 Gew.-% |
| Decandioldimethacrylat | 8.4 Gew.-% |
| Gequollener, vernetzter PMMA (Polymergel) | 20.0 Gew.-% |
| pyrogene Kieselsäure, silanisiert | 37.0 Gew.-% |
| Tert.-Butylperoctoat | 0.5 Gew.-% |

### Versuchskomposit 2:

| | |
|---|---|
| UDMA Monomer (UDMA 1) | 34.1 Gew.-% |
| Decandioldimethacrylat | 8.4 Gew.-% |
| gequollener vernetzter PMMA (Polymergel) | 20.0 Gew.-% |
| pyrogene Kieselsäure silanisiert | 37.0 Gew.-% |
| Tert.-Butylperoctoat | 0.5 Gew.-% |

Beide Komposite wurden im Autoklaven bei 140°C und einem Druck von 5 bar für 20 Minuten in Stickstoffatmosphäre auspolymerisiert.

Für den Vergleich der erfindungsgemäßen Komposite mit vergleichbaren Materialien aus dem Stand der Technik, die für Kunststoffzähne verwendet werden, wurden nachstehende Materialien verwendet:

### Konventionelles Komposit:

| | |
|---|---|
| 60 Gew.-% | UDMA 1 (mit 1 % Katalysator) |
| 40 Gew.-% | pyrogene Kieselsäure |

Vernetztes PMMA
Standardwerkstoff für Prothesenzähne)

### Physikalische Werte:

| | Beispiel 1 | Beispiel 2 | Konventionelles Komposit | Vernetztes PMMA |
|---|---|---|---|---|
| Biegefestigkeit MPa | 123±7 | 128 ± 16 | 95 | 120 |
| E-Modul MPa | 6087 ± 290 | 5550 ± 535 | 5100 | 3000 |
| Vickershärte | 380 ± 2 | 370 ± 8 | 300 ± 9 | 190 ±5 |
| Kugeldruckhärte | 310 ± 3 | 302 ± 5 | 270 ± 4 | 170±4 |

### Bestimmung der Verfärbungsneigung:

Zur Bestimmung der Verfärbungsneigung der o. g. Beispiele wurden Prüfkörper in einer Farbstofflösung (0.01%iger wässriger Safraninrotlösung) eingelagert und die Farbwerte bestimmt.

### Prüfmethode:

Die Rückflussapparatur bestand aus einem 200 ml-Rundkolben mit Glasschliff und Kugelkühler. Die o. g. Farbstofflösung (100ml) und die Prüfkörper wurden bei Raumtemperatur eingebracht, anschließend wurde die Lösung aufgeheizt und 16 Stunden unter Rückfluss kochen gelassen.

Danach wurde die Lösung zusammen mit den Prüfkörpern abgekühlt, die Proben aus dem Kolben entnommen, mit kaltem Leitungswasser abgespült und mit einem Papiertuch getrocknet. Mit einem Minolta-Farbspektrometer wurde die Differenz der Farbwerte vor und nach der Lagerung in Safraninlösung bestimmt und gegenübergestellt:

### Messwerte

| | L* | a* | b* |
|---|---|---|---|
| Vernetztes PMMA | 0.82 | -0.12 | 0.08 |
| Konventionelles Komposite | -29.56 | 43.86 | 7.34 |
| Beispiel 1 | -0.31 | 6.83 | 1.18 |
| Beispiel 2 | -1.91 | 5.35 | -1.43 |

### Resultate aus dem Verfärbungstest:

Anhand der farbmetrischen Daten lässt sich erkennen, dass Komposite, die erfindungsgemäß hergestellt werden, deutlich weniger zu Verfärbungen neigen als konventionelle Komposite. Das Beispiel 2 ist mit der gleichen Monomermatrix hergestellt wie das konventionelle Komposit, trotzdem zeigt dieses eine stark reduzierte Farbstoffaffinität.

### Bestimmung der Belagszahl:

Neben der Bestimmung der Verfärbungsneigung kann auch die Belagsanfälligkeit, die ein Material aufweist, mittels eines In-vivo-Tests nachgewiesen werden. Dazu werden in Prothesen backenseitig jeweils 3 kleine Blöcke mit bis zu neun zu testenden Materialien eingearbeitet. Da die Plaqueablagerungen von Patient zu Patient und auch von der Position im Munde verschieden sind, muss in jeden Block eine Positiv- und eine Negativprobe eingearbeitet sein. Als Referenzmaterialien wird jeweils als Negativprobe ein konventionelles Komposit (ergibt viel Belag) und als Positivprobe ein vernetztes PMMA (wenig belaganfällig) verwendet. Nach einer Tragzeit von vorzugsweise etwa einem Jahr werden die Materialien auf den Plaquebefall getestet. Dazu werden die Proben aus der Prothese herauspräpariert und unter dem Auflichtmikroskop von mindestens 3 Personen beurteilt. Anhand der Positiv- und Negativprobe kann über eine Zahlzuordnung die Belagszahl festgelegt werden. Ist die ganze Oberfläche einer Probe mit Plaque überzogen, so ergibt sich die Belagszahl 5, ist kein Belag auf der Probe, so ist die Belagszahl 0. Über Referenzproben kann zusätzlich die Plaqueneigung des Patienten bestimmt werden. Die Erfahrungen zeigen, dass ein Material mit einer Belagszahl von kleiner als 1.5 eine sehr geringe Plaqueaffinität aufweist.

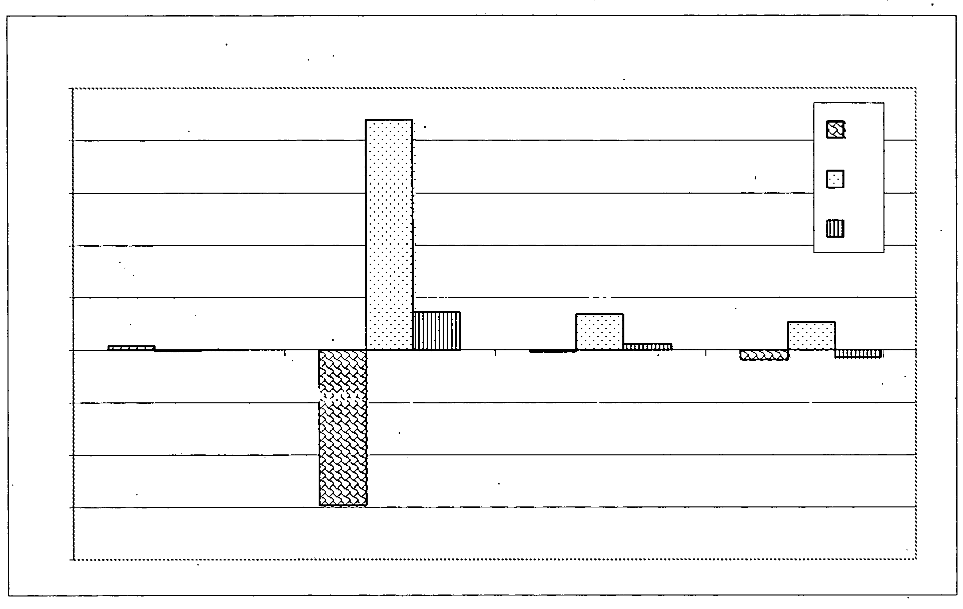

## Patentansprüche

1. Verfahren zur Herstellung von im Dentalbereich einsetzbaren Kompositen, bei welchen
a) in einem Lösungsmittel gelöste mono- oder polyfunktionelle Monomere zu einem mit dem Lösungsmittel durchquollenen Polymergel polymerisiert werden,
b) das entstandene Polymergel vorzerkleinert,
c) anschließend ein Reaktivgel hergestellt wird, indem das vorzerkleinerte Polymergel unter gleichzeitigem Entfernen des Lösungsmittels ein polymerisierbares Monomer oder eine polymersierbare Monomermischung in das Polymergel eingearbeitet und
d) aus dem so erhaltenen Reaktivgel ein Dentalkomposit hergestellt wird.

2. Verfahren nach Anspruch 1, **dadurch gekennzeichnet, dass** in Schritt a) als Monomer Methylmethacrylat, Ethylmethacrylat, Benzylmethacrylat, Isobutylmethacrylat, oder andere niedermolekulare Monomethacrylate eingesetzt werden.

3. Verfahren nach einem der vorhergehenden Ansprüche, **dadurch gekennzeichnet, dass** in Schritt a) mehrfunktionelle Monomere eingesetzt werden.

4. Verfahren nach einem der vorhergehenden Ansprüche, **dadurch gekennzeichnet, dass** in Schritt a) als Vernetzer ein Urethandimethacrylat eingesetzt wird.

5. Verfahren nach einem der vorhergehenden Ansprüche, **dadurch gekennzeichnet, dass** in Schritt a) ein nicht an der Polymerisatiorisreaktion beteiligtes Lösungsmittel eingesetzt wird.

6. Verfahren nach einem der vorhergehenden Ansprüche, **dadurch gekennzeichnet, dass** als Lösungsmittel Aceton, Ethanol, Hexan, Toluol, Ethylacetat oder ein Gemisch eines oder mehrerer dieser Lösungsmittel eingesetzt wird.

7. Verfahren nach einem der vorhergehenden Ansprüche,
**dadurch gekennzeichnet, dass** in Schritt a) 50 bis 70 Gew.-% Monomere, 95 bis 30 Gew.-% Vernetzer, 30 bis 70 Gew.-% Lösemittel eingesetzt werden, wobei die Zusammensetzung der einzelnen Komponenten sich jeweils zu 100 Gew.-% addiert.

8. Verfahren nach einem der vorhergehenden Ansprüche,
**dadurch gekennzeichnet, dass** die Polymerisation im Bereich des Siedepunktes des Lösungsmittel durchgeführt wird.

9. Verfahren nach einem der vorhergehenden Ansprüche,
**dadurch gekennzeichnet, dass** die Polymerisation in Gegenwart eines Katalysators durchgeführt wird.

10. Verfahren nach einem der vorhergehenden Ansprüche,
**dadurch gekennzeichnet, dass** als Katalysator Lauroylperoxid eingesetzt wird.

11. Verfahren nach einem der vorhergehenden Ansprüche,
**dadurch gekennzeichnet, dass** derart polymerisiert wird, dass ein klebfreies Polymergel erhalten wird.

12. Verfahren nach einem der vorhergehenden Ansprüche,
**dadurch gekennzeichnet, dass** die Vorzerkleinerung in einem Zerhacker durchgeführt wird.

13. Verfahren nach einem der vorhergehenden Ansprüche,
**dadurch gekennzeichnet, dass** das Verhältnis von Monomeren und Vernetzern zu Lösungsmitteln von 1:3 bis 1:0,75, liegt.

14. Verfahren nach einem der vorhergehenden Ansprüche,
**dadurch gekennzeichnet, dass** als polymerisierbares Monomer Urethandimethacrylat, BisGMA, Carbonatdimethacrylate, Ethylenglycoldimethacrylate oder Epoxidharze eingesetzt werden.

15. Verfahren nach einem der vorhergehenden Ansprüche,
**dadurch gekennzeichnet, dass** die Einarbeitung des vorzerkleinerten Polymergels in ein Monomer in einem Dreiwalzenstuhl erfolgt.

16. Verfahren nach einem der vorhergehenden Ansprüche,
**dadurch gekennzeichnet, dass** das vorzerkleinerte Polymergel mit kurzkettigen Vernetzern zu Vor-Pasten verarbeitet wird.

17. Verfahren nach Anspruch 16,
**dadurch gekennzeichnet, dass** die Vor-Pasten in Komposite eingearbeitet werden.

18. Verfahren nach einem der vorhergehenden Ansprüche,
**dadurch gekennzeichnet, dass** in Schritt d) für die Herstellung der Komposite anorganische oder organische Füllstoffe eingesetzt werden.

19. Verfahren nach Anspruch 18,
**dadurch gekennzeichnet, dass** als anorganische Füllstoffe SiO₂ oder Glas eingesetzt werden.

20. Komposite hergestellt in einem Verfahren nach einem der Ansprüche 1 bis 19.

21. Verwendung der Komposite nach Anspruch 20 im Dentalbereich.

## Claims

1. Process for the preparation of composites which can be used in the dental field, in which
a) mono- or polyfunctional monomers dissolved in a solvent are polymerized to give a polymer gel thoroughly swollen with the solvent,
b) the polymer gel produced is subjected to preliminary size reduction,
c) a reactive gel is subsequently prepared by incorporating a polymerizable monomer or a mixture of polymerizable monomers in the polymer gel while simultaneously removing the solvent, and
d) a dental composite is prepared from the reactive gel thus obtained.

2. Process according to Claim 1, **characterized in that**, in stage a), methyl methacrylate, ethyl methacrylate, benzyl methacrylate, isobutyl methacrylate or other low molecular weight monomethacrylates are used as monomer.

3. Process according to either of the preceding claims, **characterized in that**, in stage a), polyfunctional monomers are used.

4. Process according to one of the preceding claims, **characterized in that**, in stage a), a urethane dimethacrylate is used as crosslinking agent.

5. Process according to one of the preceding claims, **characterized in that**, in stage a), use is made of a solvent which is not involved in the polymerization reaction.

6. Process according to one of the preceding claims, **characterized in that** use is made, as solvent, of acetone, ethanol, hexane, toluene, ethyl acetate or a mixture of two or more of these solvents.

7. Process according to one of the preceding claims, **characterized in that**, in stage a), 50 to 70% by weight of monomers, 95 to 30% by weight of crosslinking agent and 30 to 70% by weight of solvent are used, the composition of the individual components in each case adding up to 100% by weight.

8. Process according to one of the preceding claims, **characterized in that** the polymerization is carried out in the region of the boiling point of the solvent.

9. Process according to one of the preceding claims, **characterized in that** the polymerization is carried out in the presence of a catalyst.

10. Process according to one of the preceding claims, **characterized in that** lauroyl peroxide is used as catalyst.

11. Process according to one of the preceding claims, **characterized in that** polymerization is carried out in such a way that a tack-free polymer gel is obtained.

12. Process according to one of the preceding claims, **characterized in that** the preliminary size reduction is carried out in a chopping device.

13. Process according to one of the preceding claims, **characterized in that** the ratio of monomers and crosslinking agents to solvents is from 1:3 to 1:0.75.

14. Process according to one of the preceding claims, **characterized in that** use is made, as polymerizable monomer, of urethane dimethacrylate, Bis-GMA, carbonate dimethacrylates, ethylene glycol dimethacrylates or epoxide resins.

15. Process according to one of the preceding claims, **characterized in that** the incorporation of the polymer gel which has been subjected to preliminary size reduction in a monomer is carried out in a three-roll mill.

16. Process according to one of the preceding claims, **characterized in that** the polymer gel which has been subjected to preliminary size reduction is processed with short-chain crosslinking agents to give prepastes.

17. Process according to Claim 16, **characterized in that** the prepastes are incorporated in composites.

18. Process according to one of the preceding claims, **characterized in that**, in stage d), inorganic or organic fillers are used for the preparation of the composites.

19. Process according to Claim 18, **characterized in that** SiO₂ or glass are used as inorganic fillers.

20. Composites, prepared in a process according to one of Claims 1 to 19.

21. Use of the composites according to Claim 20 in the dental field.

## Revendications

1. Procédé pour la préparation de composites utilisables dans le domaine dentaire, dans lequel
a) des monomères monofonctionnels ou polyfonctionnels dissous dans un solvant sont polymérisés en un gel de polymère gonflé par le solvant,
b) le gel de polymère formé est prébroyé,
c) un gel réactif est ensuite préparé en ce qu'on incorpore, dans le gel de polymère, avec élimination simultanée du solvant, un monomère polymérisable ou un mélange de monomères polymérisable et
d) un composite dentaire est préparé à partir du gel réactif ainsi obtenu.

2. Procédé selon la revendication 1, **caractérisé en ce qu'**on utilise, dans l'étape a), comme monomère, le méthacrylate de méthyle, le méthacrylate d'éthyle, le méthacrylate de benzyle, le méthacrylate d'isobutyle ou d'autres monométhacrylates de bas poids moléculaire.

3. Procédé selon l'une quelconque des revendications précédentes, **caractérisé en ce qu'**on utilise, dans l'étape a), des monomères polyfonctionnels.

4. Procédé selon l'une quelconque des revendications précédentes, **caractérisé en ce qu'**on utilise, dans l'étape a), comme réticulant, un diméthacrylate d'uréthane.

5. Procédé selon l'une quelconque des revendications précédentes, **caractérisé en ce qu'**on utilise, dans l'étape a), un solvant ne participant pas à la réaction de polymérisation.

6. Procédé selon l'une quelconque des revendications précédentes, **caractérisé en ce qu'**on utilise, comme solvant, l'acétone, l'éthanol, l'hexane, le toluène, l'acétate d'éthyle ou un mélange de deux ou de plusieurs de ces solvants.

7. Procédé selon l'une quelconque des revendications précédentes, **caractérisé en ce qu'**on utilise, dans l'étape a), 50 à 70% en poids de monomères, 95 à 30% en poids de réticulant, 30 à 70% en poids de solvant, la composition de la somme des différents composants valant à chaque fois 100% en poids.

8. Procédé selon l'une quelconque des revendications précédentes, **caractérisé en ce que** la polymérisation est réalisée dans la plage du point d'ébullition du solvant.

9. Procédé selon l'une quelconque des revendications précédentes, **caractérisé en ce que** la polymérisation est réalisée en présence d'un catalyseur.

10. Procédé selon l'une quelconque des revendications précédentes, **caractérisé en ce qu'**on utilise, comme catalyseur, du peroxyde de lauroyle.

11. Procédé selon l'une quelconque des revendications précédentes, **caractérisé en ce qu'**on polymérise de manière à obtenir un gel de polymère non adhésif.

12. Procédé selon l'une quelconque des revendications précédentes, **caractérisé en ce que** le prébroyage est réalisé dans un hacheur.

13. Procédé selon l'une quelconque des revendications précédentes, **caractérisé en ce que** le rapport de monomères et réticulants à solvants vaut 1:3 à 1:0,75.

14. Procédé selon l'une quelconque des revendications précédentes, **caractérisé en ce qu'**on utilise, comme monomère polymérisable, le diméthacrylate d'uréthane, le BisGMA, des carbonates-diméthacrylates, des diméthacrylates d'éthylèneglycol ou des résines époxydes.

15. Procédé selon l'une quelconque des revendications précédentes, **caractérisé en ce que** l'incorporation du gel de polymère prébroyé dans un monomère est réalisé dans un broyeur à trois cylindres.

16. Procédé selon l'une quelconque des revendications précédentes, **caractérisé en ce que** le gel de polymère prébroyé est transformé en précurseurs de pâtes avec des réticulants à courte chaîne.

17. Procédé selon la revendication 16, **caractérisé en ce que** les précurseurs de pâtes sont incorporés dans les composites.

18. Procédé selon l'une quelconque des revendications précédentes, **caractérisé en ce qu'**on utilise, dans l'étape d), pour la préparation du composite, des charges inorganiques ou organiques.

19. Procédé selon la revendication 18, **caractérisé en ce qu'**on utilise comme charges inorganiques, du SiO₂ ou du verre.

20. Composite préparé dans un procédé selon l'une quelconque des revendications 1 à 19.

21. Utilisation du composite selon la revendication 20 dans le domaine dentaire.
